# EUROPEAN PATENT SPECIFICATION

(11) **EP 2 011 468 B1**
(45) Date of publication and mention of the grant of the patent: **21.09.2011**
(21) Application number: 07013219.6
(22) Date of filing: 05.07.2007
(51) Int. Cl.: A61K 6/00, A61K 6/083

(54) **Dental Cement**
Zahnzement
Ciment dentaire

(43) Date of publication of application: 07.01.2009
(73) Proprietor: GC Corporation, Tokyo 174-8585 (JP)
(72) Inventor: Tokui, Hideki, Itabashi-ku Tokyo 174-8585 (JP); Yarimizu, Hideki, Itabashi-ku Tokyo 174-8585 (JP); Ota, Daisuke, Itabashi-ku Tokyo 174-8585 (JP); Mori, Takuya, Itabashi-ku Tokyo 174-8585 (JP); Nakaseko, Hisashi, Itabashi-ku Tokyo 174-8585 (JP)
(74) Representative: Müller-Boré & Partner Patentanwälte

(56) References cited:
- WO-A-2004/100900
- US-B1- 6 291 548
- US-B1- 6 369 164

## Description

The present invention relates to a dental cement used for tooth restoration and the like.

Heretofore a zinc phosphate cement, a carboxylate cement, a glass ionomer cement, and a resin cement are widely used as a dental cement. In these cements, the use frequency of the zinc phosphate cement decreases because the zinc phosphate cement has no adhesiveness to tooth and it might cause stimulation for tooth in the initial curing caused by low pH value by including phosphoric acid. The carboxylate cement has low stimulation for tooth, however, its mechanical strength is low and the carboxylate cement lacks reliability.

The glass ionomer cement is used by making polycarboxylic acid react with fluoroaluminosilicate glass powder under existence of water to be cured. The glass ionomer cement has excellent biocompatibility for a living body, excellent aesthetic property since a cured cement is translucent, excellent adhesive force to tooth structure such as enamel and dentin, and an anticariogenic action due to gradually releasing fluoride ion from fluoroaluminosilicate glass. Thus, in a dental field, the dental glass ionomer cement has widely been used. However, glass ionomer cement has the character of low flexural strength and brittleness compared with the resin cement. On another front, the resin cement is excellent in mechanical strength, although, it has no adhesiveness to the tooth.

Tooth structure flexural solubility. In order to solve the problems of the glass ionomer cement, such as being more brittle than resin-based cement, particularly low flexural strength and high solubility after curing, a resin-modified glass ionomer cement in which a polymerizable monomer such as a (meth) acrylate monomer or the like is blended as a resin component has been developed (for example, refer to Japanese Patent Applications Laid Open Nos. 8-026925, 9-255515, and 6-070088).

However, such a resin-modified glass ionomer cement has a problem that hydroscopic expansion is large. The reason of this problem is as follows. Since the resin-modified glass ionomer cement includes polycarboxylic acid, water, and a polymerizable monomer being hardly dissolved in water as a liquid agent, it is necessary to blend a polymerizable monomer having a hydroxyl group, a molecular weight of less than 160, and high hydrophilicity in order to compatibilize these components of the resin-modified glass ionomer cement. For example, 2-hydroxyethylmethacrylate is used as such a polymerizable monomer. Since this polymerizable monomer such as 2-hydroxyethylmethacrylate or the like has remarkably high hydrophilicity due to the molecule structure, a cured cement absorbs water in an oral cavity so as to be expanded. When a ceramics crown having a low mechanical strength is used as a dental prosthesis, the cured cement expanded by absorbing water may break the dental prosthesis due to swelling stress. Thus, there is a problem that the conventional resin-modified glass ionomer cement cannot be used for a ceramics crown having a low mechanical strength.

The present inventors have proposed a dental cement in which a liquid agent including 4-methacryloxyethyltrimellitic acid and water is used without using polycarboxylic acid and 2-hydroxyethylmethacrylate (refer to Japanese Patent ApplicationLaidOpenNo. 2000-053518). However, this dental cement has the high blending ratio of 4-methacryloxyethyltrimellitic acid having an acid group so as to generate much salts including metal ions at the initial time of a curing reaction, where the metal ions are caused from metal oxide powder, which is a powder agent and include a fluoroaluminosilicate glass powder or zinc oxide as a main component. Such a salt gradually dissolves in an aqueous solution so that there is a problem that the solubility of the cured cement is made high.

WO 2004/100900 A1 describes dental adhesive compositions and methods.

An objective of the present invention is to provide a dental cement having a high mechanical strength like the resin-modified dental glass ionomer and the resin cement, adhesiveness to tooth, low hydroscopic expansion and resin-modified tooth structure solving a fault of high solubility.

The earnest work was carried out in order to solve the above-mentioned problems and, as a result of this, present inventors found out the followings to complete the present invention. When a liquid agent is prepared by blending a (meth)acrylate monomer having an acid group, a (meth) acrylate monomer not having an acid group and having a specified molecular weight, and a proper polymerization initiator for a polymerization catalyst in a powder agent, the liquid agent can be well prepared without blending a polymer having an acid group such as polycarboxylic acid, and 2-hydroxyethylmethacrylate.

In particular, the present invention is a dental cement comprising:
a liquid agent including
   5 to 50 % by weight of (meth)acrylate monomer having an acid group,
   Optionally 0.1 to 25 % by weight of water,
   30 to 85 % by weight of (meth) acrylate monomer having two or less hydroxyl groups and/or amino groups, not having an acid group, and having a molecular weight of 160 or more, and
   0.01 to 5 % by weight of an amine compound as a polymerization initiator for a polymerization catalyst in a powder agent described below; and
a powder agent including
   90 to 99.8 % by weight of metal oxide powder used for fluoroaluminosilicate glass powder, dental zinc phosphate cement powder, or dental carboxylate cement powder, and
   0.01 to 5 % by weight of the total of an organic aromatic compound containing at least one -SO₂- group and peroxide as a polymerization catalyst.

The dental cement according to the present invention has high adhesiveness to tooth structure as well as high mechanical strength as in the resin-modified dental glass ionomer cement and the resin cement, gives low hydroscopic expansion, and can reduce the solubility of the cured cement. Thus, this dental cement is excellent.

A (meth)acrylate monomer having an acid group, which is a constitution component in the dental cement according to the present invention, gives high adhesiveness to tooth structure and forms a dental cement matrix by polymerizing and curing with a (meth)acrylate monomer not having an acid group and having a specified molecular weight, which is the other constitution element of the dental cement of the present invention. When the blending ratio of this (meth)acrylate monomer having an acid group is less than 5 % by weight with respect to the liquid agent, adhesiveness of the dental cement to tooth structure is low. When the blending ratio of this (meth) acrylate monomer having an acid group is more than 50 % by weight, salts by reaction with metal ions at the initial stage of curing form much so that a cured cement has a high solubility.

A (meth)acrylate monomer having an acid group, which is a constitution component in the dental cement according to the present invention, preferably has a phosphoric acid group or a carboxyl group as the acid group. Since the phosphoric acid group has stronger acidity than the carboxyl group, the polymerizable monomer having the phosphoric acid group has excellent effects to dissolve a smear layer of a tooth surface and decalcify dentin, and especially exercises an excellent improvement of the adhesive property to enamel. The polymerizable monomer having a phosphoric acid group has one or a plurality of phosphoric acid groups in one molecule, and 2-(meth) acryloyloxyethyl dihydrogen phosphate, bis(meth) acryloxyethyl phosphate, bis[2- (meth)acryloyloxyethyl] hydrogen phosphate, 2-(meth) acryloyloxyethylphenyl hydrogen phosphate, acid phosphoxyethyl(meth) acrylate, 6-(meth)acryloyloxyhexyl dihydrogen phosphate, 6-(meth)acryloyloxyhexylphenyl hydrogen phosphate, 10-(meth)acryloyloxydecyl dihydrogen phosphate, 1,3 -di(meth)acryloylpropane-2-dihydrogenphosphate, 1,3 -di(meth)acryloylpropane-2-phenyl hydrogen phosphate, bis[5-{2-(meth) acryloyloxyethoxycarbonyl} heptyl] hydrogen phosphate, the reaction product obtained by reacting an additional polymer of 6-hexanolide of 2-hydroxyethyl(meth) acrylate with anhydrous phosphoric acid, and the like can be used. Among these monomers, 10-(meth)acryloyloxydecyl dihydrogen phosphate is especially preferable from the point of its adhesiveness and stability of a monomer itself. These polymerizable monomers having a phosphoric acid group can be used independently or by mixing two or more.

As the polymerizable monomer having a carboxyl group, 4-(meth) acryloxyethyltrimellitic acid, 4-(meth)acryloxyethyltrimellitic acid anhydride, 4-(meth)acryloxydecyltrimellitic acid, 4-(meth) acryloxydecyltrimellitic acid anhydride, 11- (meth)acryloyloxy-1,1-undecanedicarboxylic acid, 1, 4 di(meth)acryloyloxypyromellitic acid, 2- (meth) acryloyloxyethylmaleic acid, 2- (meth)acryloyloxyethylphthalic acid, 2-(meth) acryloyloxyethylhexahydrophthalic acid, and the like can be used. Among these monomers, 4-(meth) acryloxyethyltrimellitic acid and 4- (meth)acryloxyethyltrimellitic acid anhydride are especially preferable from the point of adhesiveness.

When a monomer having a carboxyl group, for example, 4-(meth)acryloxyethyltrimellitic acid anhydride is used as the (meth) acrylate monomer having an acid group, the monomer is preferably used in the form of an aqueous solution so that storage stability is improved. Therefore, 0.1 to 25 % by weight of water is preferably blended with the liquid agent of the cement. When the blending ratio of water is less than 0.1 % by weight, the effect of improvement of storage stability of the (meth) acrylate monomer having an acid group is hardly obtained. When the blending ratio is more than 25 % by weight, the mechanical strength of the cured cement, more particularly, the flexural strength may decrease.

The (meth)acrylate monomer having two or less hydroxyl groups and/or amino groups, not having an acid group, and having a molecular weight of 160 or more is used as a constitutional component of the liquid agent in the dental cement according to the present invention. Since this monomer has few portions of a hydrophilic group with respect to the molecular weight, the cured cement after polymerization hardly absorbs water and, as a result of this, the cured cement hardly swells. Thus, the (meth)acrylate monomer can be used for a ceramics crown type prosthesis having low strength. Further, the cured cement after polymerization is more stable in water, and thus solubility can decrease. Further, the (meth)acrylate monomer has an effect to increase mechanical strength, more particularly, flexural strength of the cured cement. When the molecular weight is less than 160, the portions of a hydrophilic group become too large with respect to the molecular weight. Thus, since the cured cement after polymerization easily swells by absorbing water, it is not proper. Furthermore, even when the molecular weight is more than 160, the ratio of a hydrophilic group becomes too high when the monomer has three or more hydroxyl groups and/or amino groups per molecule. Thus, since the cured cement after polymerization easily swells by absorbing water, it is not proper.

As for the blending ratio of the (meth)acrylate monomer having two or less hydroxyl groups and/or amino groups, not having the acid group and having the molecular weight of 160 or more, when the blending ratio of the monomer in the liquid agent is less than 30 % by weight, the above-described effect cannot be obtained. When the blending ratio is more than 85 % by weight, adhesiveness to tooth structure decreases.

As the (meth)acrylate monomer having two or less hydroxyl groups and/or amino groups, not having the acid group, and having the molecular weight of 160 or more, many monomer conventionally used in a dental field can be used. For example, benzyl(meth)acrylate, 2,2-bis[(meth) acryloxyphenyl] propane, 2,2-bis[4-(meth)acryloxydiethoxyphenyl] propane, 2,2-bis[4-(meth)acryloxypolyethoxyphenyl] propane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth) acrylate, 1,6-hexanediol di(meth)acrylate, trimethylolpropane tri(meth)acrylate, pentaerythritoltri(meth)acrylate, trimethylolmethane tri(meth)acrylate, pentaerythritol tetra(meth)acrylate, 2- hydroxyethyl(meth) acrylate, 2- hydroxypropyl(meth)acrylate, 2-hydroxy-1,3-di(meth) acryloxypropane, 1,2-dihydroxy-3- (meth)acryloxypropane, 2,2-bis[4-{2-hydroxy-3-(meth)acryloxypropoxy} phenyl] propane can be used. Further, as the polymerizable monomer not having an acidic group, which has urethane bond in a molecule, di-2-(meth)acryloxyethyl-2,2,4 -trimethylhexamethylene dicarbamate or the like, can be used.

As the amine compound which is a component of the liquid agent in the dental cement according to the present invention and used as a polymerization initiator for a polymerization catalyst in a powder agent, an aromatic tertiary amine and an aliphatic tertiary amine and the like are effective. Particularly, N,N-dimethyl-p-toluidine, N,N-diethyl-p-toluidine, N,N-dimethylaniline, N,N-bis (2- hydroxyethyl)-p-toluidine, N,N-dimethylaminoethylmethacrylate, triethanolamine, 4-methyl dimethylamino benzoate, 4-ethyl dimethylamino benzoate, 4-isoamyl dimethylamino benzoate, triethylamine, N-ethyl diethanol amine, triethanol amine, and the like can be used. Of course, these amine compounds can be used independently or by mixing two or more kinds.

The liquid agent necessarily includes 0.01 to 5 % by weight of amine compounds. When a blending ratio is less than 0.01 % by weight, it is insufficient to have an ability of the polymerization initiator for a polymerization catalyst in a powder agent. When the ratio is more than 5 % by weight, the cured cement may be discolored and the effect is hardly increased.

The powder agent in the dental cement according to the present invention is mainly to compose a filler component of the dental cement. As the filler component, metal oxide powder which can be cured by a cement reaction (an acid-base reaction) in the liquid agent including a (meth) acrylate monomer having an acid group and water, and is used for the fluoroaluminosilicate glass powder, dental zinc phosphate cement powder, or dental carboxylate cement powder is preferable.

This fluoroaluminosilicate glass powder is a glass powder conventionally used for a dental glassionomer cement and preferably includes Al³⁺, Si⁴⁺, F⁻, O²⁻, and further Sr²⁺ and/or Ca²⁺ as main components. As for the ratios of the components with respect to the whole amount of the glass, it is preferable to include 10 to 21 % by weight of Al³⁺, 9 to 24 % by weight of Si⁴⁺, 1 to 20 % by weight of F⁻, and 10 to 34 % by weight of the total of Sr²⁺ and Ca²⁺. The ratios of the main components much affect operativity and physical properties, such as a curing speed, final strength, solubility and the like. When the ratio of Al³⁺ is less than 10 % by weight, curing is insufficient, and the strength is also low. When the ratio of Al³⁺ is more than 21 % by weight, it is hard to produce a glass, so that transparency may decrease, and an aesthetic property may be inferior. When the ratio of Si⁴⁺ is less than 9 % by weight, it easily becomes hard to produce a glass. If the ratio of Si⁴⁺ is more than 24 % by weight, the curing speed easily becomes low, and the strength may decrease, so that there is a problem in durability. When the ratio of F⁻ is less than 1 % by weight, a working time of the cement when mixing the liquid agent and the powder agent is short, so that it becomes hard to use and operate the component. When the ratio of F⁻ is more than 20 % by weight, a final setting time is long, and a solubility in water is high, so that the durability may be inferior. When the total of Sr²⁺ and Ca²⁺ is less than 10 % by weight, the sharpness of curing can not be exercised and the setting time easily becomes long so that it may become hard to produce a glass. When the total of Sr²⁺ and Ca²⁺ is more than 34 % by weight, the working time is not enough since the curing speed is too high, so that it may become hard to use this component. In this case, a solubility in water increases, and a durability may decrease. The fluoroaluminosilicate glass used in the present invention can be produced by a publicly known glass producing method.

Further, dental zinc phosphate cement powder and dental carboxylate cement powder are metal oxide powder including zinc oxide as a main component. They can be generally produced by mixing 10 to 30 % by weight of a metal oxide such as magnesium oxide or the like with 70 to 90 % by weight of zinc oxide, sintering the mixture at a high temperature of 700 degree C or more, cooling, and pulverizing by a ball mill or the like. In addition, as the other metal oxide than magnesium oxide, for example, strontium oxide, silicon dioxide, ferric oxide, yttrium oxide and the like can be used.

As for metal oxide powder used for the fluoroaluminosilicate glass powder, the dental zinc phosphate cement powder and the dental carboxylate cement powder in the powder agent, it is desirable to adjust the particle size of the powder. The preferable average particle diameter is 0.02 µm to 10 µm, more preferably 0.02 µm to 5 µm. When the fine powder having the average particle diameter of less than 0.02 µm is used, the reaction speed with the (meth)acrylate monomer having an acid group in the liquid agent is too high, so that operativity may worsen. When the average particle diameter is more than 10 µm, the curing reaction is too slow, and the surface roughness of the dental cement may be more coarse. Further, the powder agent used in the dental cement composition according to the present invention can be subjected to a silane treatment by a conventional method.

As for the constitutional component of the powder agent in the dental cement according to the present invention, the peroxide and the organic aromatic compound contains at least one -SO₂- group as a polymerization catalyst, can operate as a polymerization catalyst by the operation as a polymerization initiator of amine compound in a liquid agent. However, when the organic aromatic compound containing at least one -SO₂- group is added, the polymerizability of a (meth)acrylate monomer under acidity is enhanced. The organic aromatic compound containing at least one -SO₂- group is an aromatic sulfinic acid or a metal salt of the aromatic sulfinic acid, or an aromatic sulfonyl compound. For example, sodium p-toluenesulfinate, lithium p-toluenesulfinate, benzenesulfinic acid, sodium benzenesulfinate, p-toluenesulfonyl chloride, p-toluenesulfonyl fluoride, o-toluenesulfonyl isocyanate, p-toluenesulfonyl hydrazide, p-toluene sulfonamide, p-toluenesulfonyl imidazole, p-toluenesulfonyl cyanide, 2-(p-toluenesulfonyl) acetophenone, p-toluenesulfonyl-N-diethylamide, α-N,α- toluenesulfonyl-N-arginine, α -N,p-toluenesulfonyl-L-arginine methyl ester, p-toluenesulfonylmethyl isocyanate, p-toluenesulfonyl-N-methyl-N-nitrosamide, N-(p-toluenesulfonyl)-L-phenylalanine, N-p-toluenesulfonyl-L- phenylalanyl chloride, p-toluenesulfonyl acetonitrile, 2-(p-toluenesulfonyl) acetophenone, toluene-3, 4-disulfonyl chloride, benzene sulfonamide, benzene sulufohydroxamine acid, benzenesulfonyl chloride, benzenesulfonyl isocyanate, benzene sulfonanilide, benzene sulfonchloramide sodium, benzene sulfondichloramide, benzenesulfonyl hydrazide, benzenesulfonyl-N-methylamide, 2-phenylsulfonyl acetophenone, diaminodiphenylsulfon, 4, 4'-sulfonyldiphenol, sulfapyridine, sulfaaerosol, sulfamethizole, ethylbenzenesulfonyl chloride, nitrobenzenesulfonyl chloride, nitrobenzenesulfonyl fluoride, and the like can be used. In addition, these organic aromatic compounds containing at least one -SO₂- group may be a hydrous salt.

As the peroxide, potassium peroxodisulfate, sodium peroxodisulfate, ammonium peroxodisulfate, benzoylperoxide, 4, 4'-dichlorobenzoylperoxide, 2, 4 dichlorobenzoylperoxide, dilauroylperoxide, and the like can be used. In these compounds, potassium peroxodisulfate and benzoylperoxide are preferable. These compounds can be used independently or by mixing two or more kinds.

The total of the blending amount of the organic aromatic compound containing at least one -SO₂- group and the peroxide with respect to the powder agent is 0.01 to 5 % by weight. When the amount is less than 0.01 % by weight, the ability as the polymerization catalyst is insufficient, and the polymerization of the polymerizable monomer becomes un-uniform. When the amount is more than 5 % by weight, the cured cement may be discolored even though the effect is hardly increased.

As for a mixing ratio of the liquid agent and the powder agent in the dental cement according to the present invention, it is preferable that the powder agent is 0.5 to 5 by weight with respect to 1 by weight of the liquid agent. When the ratio of the powder agent is less than 0.5 by weight, mechanical strength of the dental cement after curing may decrease. When the ratio is more than 5 by weight, it is hard to mix the powder agent with the liquid agent, and the adhesiveness to tooth structure may be inferior. More particularly, when the ratio is 1.5 to 3.8, it is preferable from the point of the viscosity after mixing.

In addition, of course, a photopolymerization catalyst, an antibacterial agent, a pigment, a stabilizer and the like can be properly blended in the dental cement according to the present invention if necessary.

### [Example]

Hereinafter, the present invention will be concretely described with examples.

### [Preparation of a fluoroaluminosilicate glass powder as a powder agent]

The blending ratios of the fluoroaluminosilicate glass powders I, II and III are shown in Table 1.

**[Table 1]**

| | Fluoroaluminosilicate Glass Powder | | |
|---|---|---|---|
| | I | II | III |
| Aluminium Oxide (g) | 21 | 23 | 22 |
| Silicic Anhydride (g) | 44 | 41 | 43 |
| Calcium Fluoride (g) | 12 | 10 | 12 |
| Calcium Phosphate (g) | 14 | 13 | 15 |
| Strontium Carbonate (g) | 9 | 13 | 8 |

The fluoroaluminosilicate glass powders I and III were produced by fully mixing materials, melting the mixture by heating in an electric furnace at 1200 degree C for 5 hours, cooling, pulverizing for 10 hours by using a ball mill, passing through a 200-mesh sieve (ASTM), adding 1g of γ-methacryloxypropyltrimethoxysilane and 9 g of ethanol with respect to 100 g of the powder, and subjecting to a dry type silane coupling treatment based on a conventional method. The fluoroaluminosilicate glass powder II was produced by the similar process to that of the fluoroaluminosilicate glass powders I and III except melting of the glass at 1,100 degree C.

### [Preparation of a metal oxide powder as a powder agent other than the fluoroaluminosilicate glass powder]

The blending ratios of the metal oxide powders I and II used for the dental zinc phosphate cement powder and the dental carboxylate cement powder are shown in Table 2.

**[Table 2]**

| | Metal Oxide Powder | |
|---|---|---|
| | I | II |
| Zinc Oxide (g) | 88 | 80 |
| Magnesium Oxide (g) | 12 | 18 |
| Strontium Oxide (g) | - | 2 |

The metal oxide powder I including zinc oxide as a main component was produced by, fully mixing materials, sintering the mixture by heating for 5 hours in an electric furnace at 1,000 degree C, cooling, pulverizing for 10 hours by using a ball mill, and passing through a sieve of 200-mesh (ASTM). The metal oxide powder II including zinc oxide as a main component was produced by the similar process to that of the metal oxide powder I except sintering of the mixture at 900 degree C.

### [Preparation of a powder agent and a liquid agent]

The blending ratios of powder agents and liquid agents used in examples and comparison examples are shown in Table 3. The powder agents were prepared by mixing a fluoroaluminosilicate glass powder or a metal oxide powder and a polymerization catalyst for 20 minutes in a mortar. The liquid agents were prepared by mixing a (meth) acrylate monomer having an acid group and water for 4 hours at a room temperature, mixing each component described in Table 3 except silicon dioxide as a filler until the mixture becomes uniform, adding the silicon dioxide as a filler, and mixing for 1 hour.

### [Table 3]

Abbreviations in Table 3 will be described as follows.
(Meth)acrylate monomers having an acid group
MDP: 10-methacryloyloxydecyl dihydrogen phosphate
PM2: bismethacryloxyethyl phosphate
PM21: a reaction product obtained by reacting an additional polymer of 6-hexanolide of 2-hydroxyethylmethacrylate with anhydrous phosphoric acid
Phosmer M: acid phosphoxyethylmethacrylate
4META: 4-methacryloxyethyltrimellitic anhydride (Meth)acrylate monomers having two or less hydroxyl groups and/or amino groups, not having an acid group and having a molecular weight of 160 or more TEGDMA: triethyleneglycoldimethacrylate
GDMA: 2-hydroxy-1,3-dimethacryloxypropane
UDMA: di-2-methacryloxyethyl-2,2,4- trimethylhexamethylene dicarbamate
Amine compound
P amine: N,N-bis(2-hydroxyethyl)-p-toluidine The other additives
SiO₂ : Silicon dioxide
BHT: 2,6-di-tert-butyl-p-cresol
CQ: camphorquinone
Metal oxide powders
Glass powder I: fluoroaluminosilicate glass powder I
Glass powder II: fluoroaluminosilicate glass powder II
Glass powder III: fluoroaluminosilicate glass powder III
Metal oxide powder I: metal oxide powder I including zinc oxide as a main component
Metal oxide powder II: metal oxide powder II including zinc oxide as a main component
Organic aromatic compounds having at least one -SO₂- group
BSA: benzenesulfinic acid
pTSNa: sodium p-toluenesulfinate/ tetrahydrate Peroxides
BP0: benzoylperoxide
KPS: potassium peroxodisulfate
NaPS: sodium peroxodisulfate

### [Tensile bond strength test]

A tooth surface for the test was obtained by grinding the surface of a bovine tooth by the water-resistant grinding paper #600 under water pouring so as to expose the surfaces of enamel and dentin. The area of the tooth surface was regulated by a plastic masking tape having a hole with a diameter of 3 mm. Then, the mixed dental cement composition was placed on the adhered surface, and a stainless rod was luted with the adhered surface by pressing by hand, where the rod had the surface ground by the water-resistant grinding paper #120 and subjected to a sandblast treatment beforehand. In the case of a dental cement composition containing a photopolymerization catalyst, an acryl rod subjected to the similar treatments to those of the above-described stainless pillar rod was used. After pressing to lute with the adhered surface, the rod was irradiated with light for 20 seconds respectively from the front, rear, left and right directions by a dental visible light curing unit (the product name wa's GC CO-BEE, produced by GC Corporation) . The specimens were kept for 1 hour in a thermostatic vessel at the relative humidity of 100% and temperature of 37 degree C, and dipped in water at temperature of 37 degree C for 23 hours. Then, the tensile bond strength was measured by using an universal test machine (the product name was Autograph, produced by Shimazu Seisakusyo Corporation) at the crosshead speed of 1.0 mm/min, so that an adhesive strength was measured.

### [Flexural strength]

A pillar shaped cured cement was obtained by filling a mixed dental cement composition into an acryl tube having an inner diameter of 3 mm and a length of 25 mm. In the case of a dental cement composition containing a photopolymerization catalyst, it was irradiated with light for 20 seconds respectively from four directions by a dental visible ray irradiator (the product name was GC CO-BEE, produced by GC Corporation). The obtained specimens were dipped in distilled water at temperature of 37 degree C for 24 hours, and the flexural strength was measured by three points flexural test by using a universal test machine (the product name was Autograph, produced by Shimazu Seisakusyo Corporation), in which a span length was 20 mm and a crosshead speed was 1.0 mm/min.

### [Hydroscopic expansion]

A cured cement was obtained by filling a mixed dental cement composition into a metal mold having the diameter of 4 mm and the height of 6 mm. In the case of a dental cement containing a photopolymerization catalyst, the cement was filled into the metal mold, and it was light cured from the top and the bottom for 20 seconds respectively by a dental visible light curing unit (the product name was GC CO-BEE, produced by GC Corporation). The specimens were taken from the mold after 24 hours, and an initial length in the height direction was measured. Then, the specimens were dipped in distilled water at 37 degree C for 24 hours, and the length in the height direction was measured. Then, the value of the initial length was subtracted from the value of the length in the height direction after dipping in distilled water for 24 hours. The balance was divided by the value of the initial length, and then multiplied by 100. The obtained value, which was a linear expansion coefficient, was determined as a hydroscopic expansion hygroscopic expansion coefficient.

### [Acid-solubility]

In order to evaluate the solubility of a dental cement composition, an acid-solubility test was carried out. A mixed dental cement composition was filled into a mold made of polymethylmethacrylate having a hole with a diameter of 5 mm and a depth of 2 mm. After pressing and sealing it with a film, the mold is held in a thermostatic vessel at the relative humidity of 100% and 37 degree C for 24 hours. In the case of a dental cement composition containing a photopolymerization catalyst, after the composition is filled into the mold and pressed with a film, the cement surface was irradiated with light for 20 seconds by a dental visible ray irradiator (the product name was GC CO-BEE, produced by GC Corporation) from the surface of the cement. Then, the mold was held in a thermostatic vessel at the relative humidity of 100% and 37 degree C for 24 hours. Then, the surface of the cured cement was ground by the water-resistant grinding paper #600 under water pouring without removing the cured cement from the mold so as to make the surface flat. The initial length between the surface and a rear face of the cured cement were measured. The test piece was dipped in 0.1 mol/L of a lactic acid/sodium lactate buffer solution (pH2.74) at 37 degree C for 24 hours, and thereafter the length of the cured cement was similarly measured. The reduced amounts of the lengths between the surface and rear face of the cured cement was evaluated.

### Examples 1 to 11

In each Example, 1.0 g of a liquid agent and 2.0 g of a powder agent were weighed and put on a kneading paper for mixing. The liquid agent and the powder agent were mixed for 40 seconds by using a spatula so as to be uniformly mixed. The results of the tensile bond strength test, the flexural strength test, the hydroscopic expansion test and the acid-solubility test of the examples are shown in Table 3.

### Comparative example 1

Polycarboxylic acid and 2-hydroxyethylmethacrylate were not used for a liquid agent of a dental glass ionomer cement, but (meth) acrylate having an acid group and water were used for a liquid agent to produce a conventional dental cement shown in the column of comparative example 1 in Table 3. 1.0 g of a liquid agent and 2.0 g of a powder agent were weighed and put on a kneading paper. The liquid agent and the powder agent were mixed by using a spatula so as to be uniformly mixed like Examples 1 to 11. The testing methods were the same as those in the examples.

### Comparative example 2

"Fuji I" (produced by GC Corporation) was used as a conventional dental glass ionomer cement. 1.0 g of a cement liquid and 1.8 g of a cement powder were weighed and put on a kneading paper. The cement liquid and the cement powder were mixed by using a spatula so as to be uniformly mixed like Examples 1 to 11. The testing methods were the same as those in the examples.

### Comparative example 3

Polycarboxylic acid and 2-hydroxyethylmethacrylate were used as a liquid agent for a conventional resin-modified dental glass ionomer cement. 1.0 g of a liquid agent and 2.0 g of a powder agent were weighed and put on a kneading paper. The liquid agent and the powder agent were mixed by using a spatula so as to be uniformly mixed like Examples 1 to 11. The testing methods were the same as those in the examples.

### Comparative example 4

"LIVCARBO" (produced by GC Corporation) was used as a conventional dental carboxylate cement. 1.0 g of a liquid agent and 2.0 g of a powder agent were weighed and put on a kneading paper. The liquid agent and the powder agent were mixed by using a spatula so as to be uniformly mixed like Examples 1 to 8. The testing methods were the same as those in the examples.

Clearly from Table 3, it was confirmed that the dental cements of Examples 1 to 11 had high flexural strength, adhesiveness to tooth structure, low hydroscopic expansion, and a low solubility.

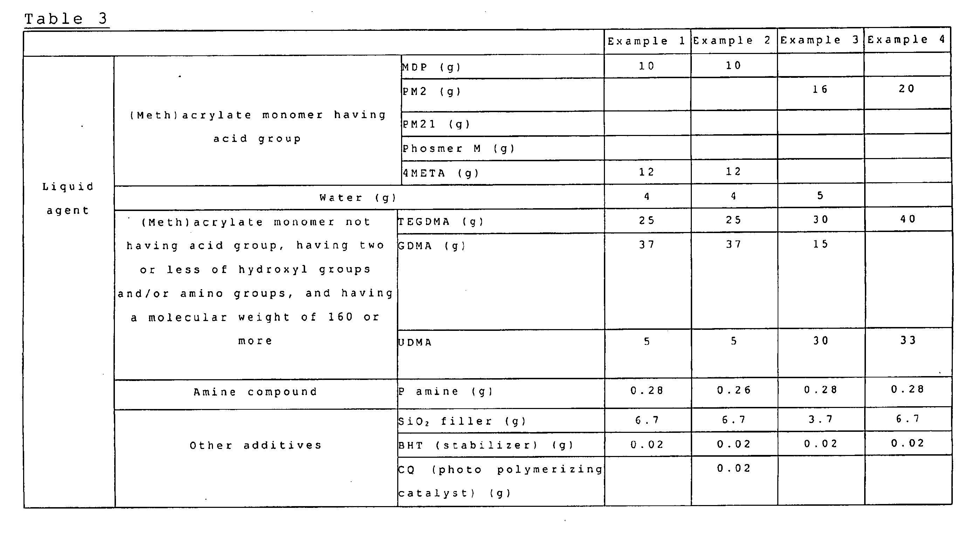

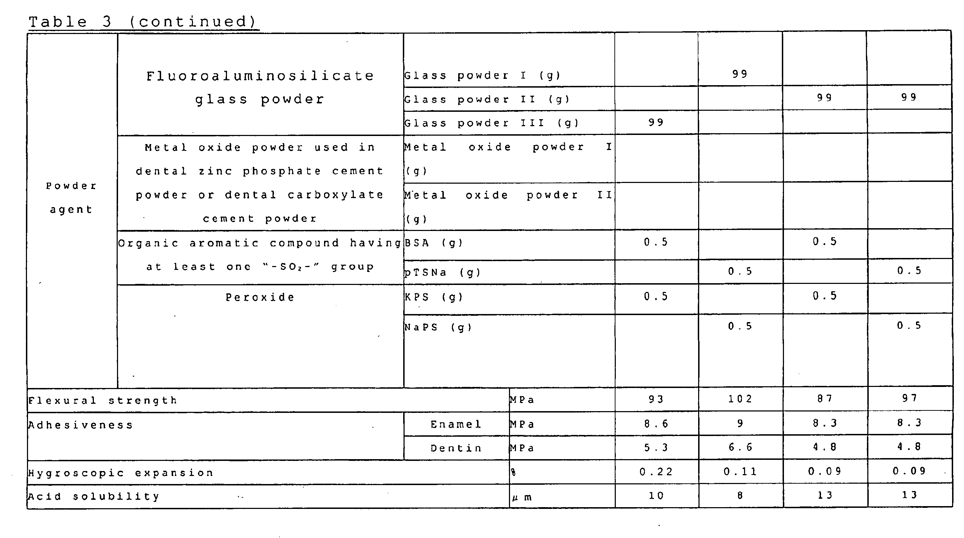

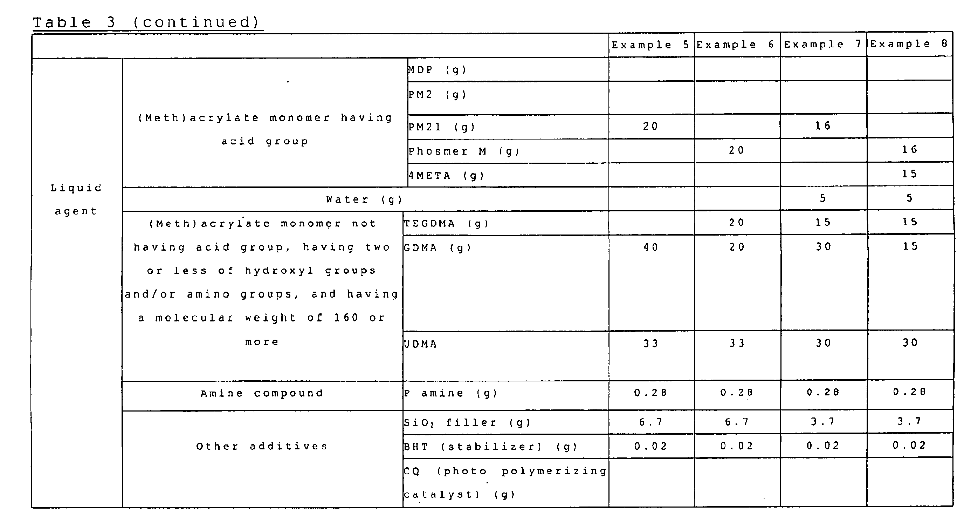

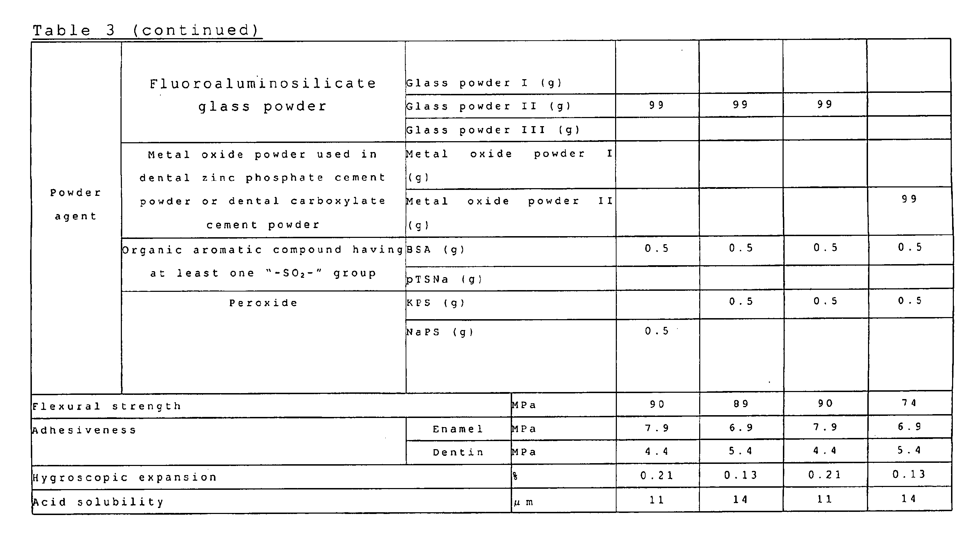

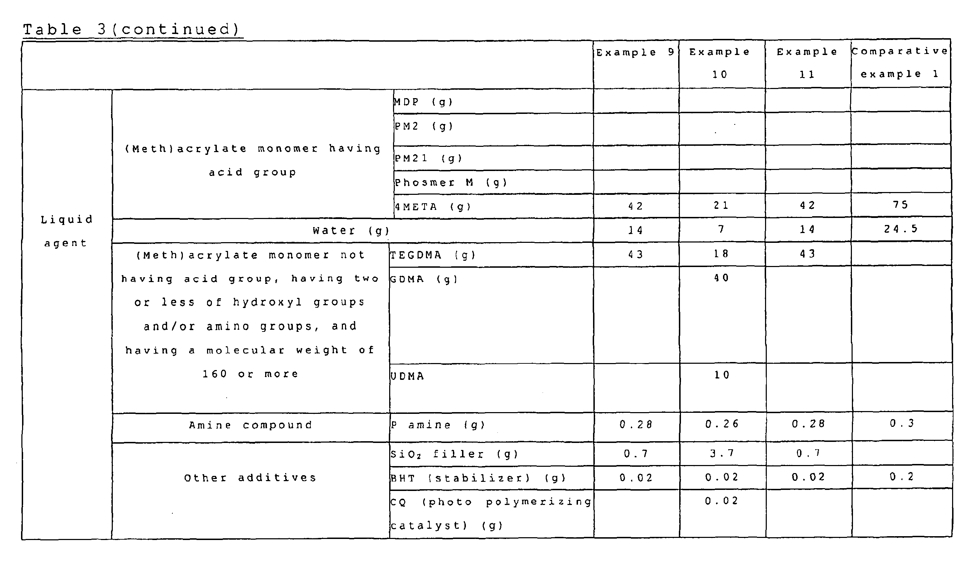

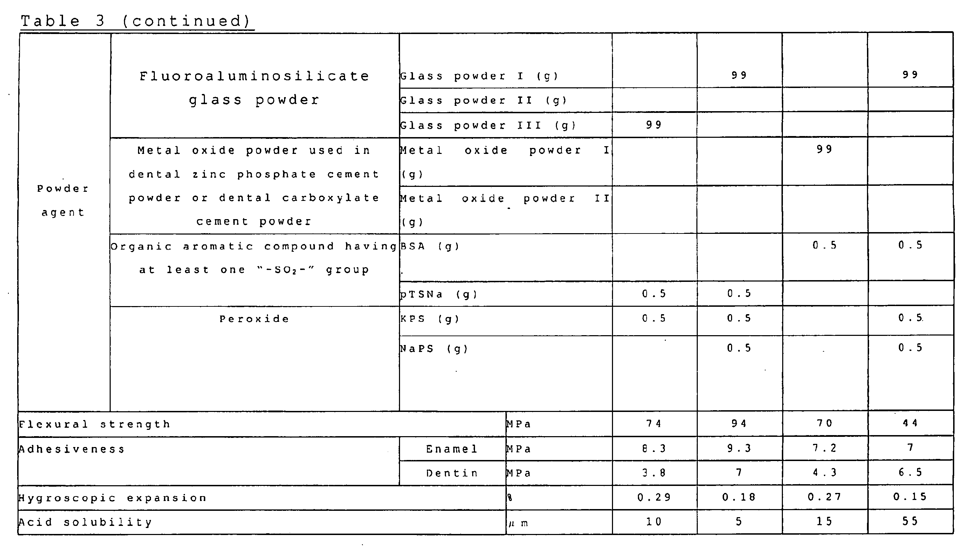

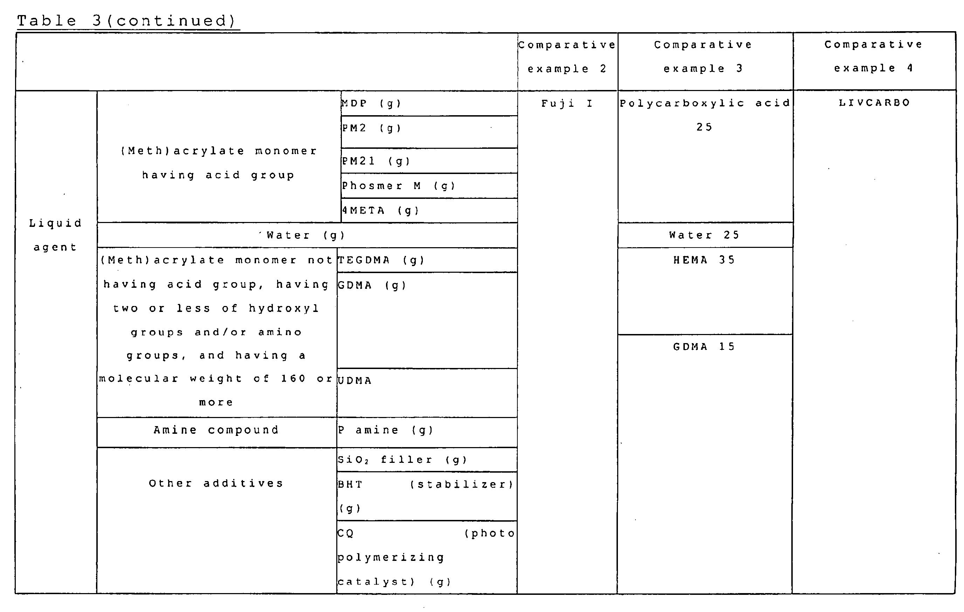

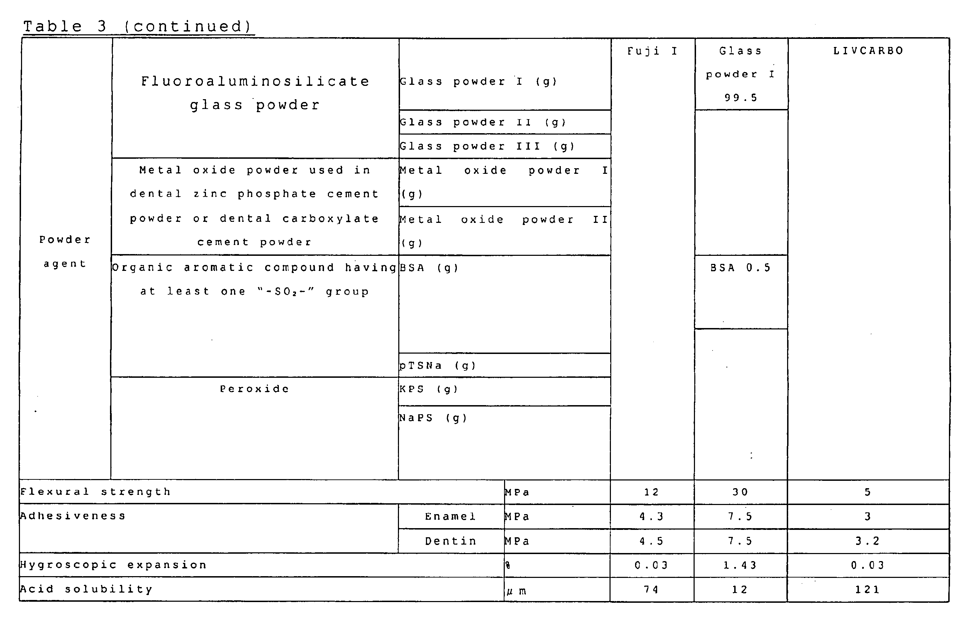

## Claims

1. A dental cement comprising:
a liquid agent comprising
5 to 50 % by weight of (meth)acrylate monomer having an acid group,
30 to 85 % by weight of (meth) acrylate monomer having two or less hydroxyl groups and/or amino groups, not having an acid group, and having a molecular weight of 160 or more, and
0.01 to 5 % by weight of an amine compound as a polymerization initiator for a polymerization catalyst in a powder agent described below; and
a powder agent comprising
90 to 99.8 % by weight of a metal oxide powder used for a fluoroaluminosilicate glass powder, a dental zinc phosphate cement powder, or a dental carboxylate cement powder, and
0.01 to 5 % by weight of the total of an organic aromatic compound containing at least one -SO₂- group and a peroxide as a polymerization catalyst.

2. The dental cement according to claim 1, wherein the liquid agent further comprises
0.1 to 25 % by weight of water.

3. The dental cement as claimed in claim 2, wherein a monomer having a carboxyl group is included as a (meth)acrylate monomer having an acid group in the liquid agent.

## Patentansprüche

1. Zahnzement, umfassend:
ein flüssiges Mittel, umfassend
5 bis 50 Gewichts-% eines (Meth)acrylat-Monomers mit einer Säuregruppe,
30 bis 85 Gewichts-% eines (Meth)acrylat-Monomers mit zwei oder weniger Hydroxylgruppen und/oder Aminogruppen, das keine Säuregruppe aufweist und ein Molekulargewicht von 160 oder mehr aufweist, und
0,01 bis 5 Gewichts-% einer Aminverbindung als ein Polymerisationsinitiator für einen Polymerisationskatalysator in einem nachstehend beschriebenen Pulvermittel; und
ein Pulvermittel, umfassend
90 bis 99,8 Gewichts-% eines Metalloxidpulvers, verwendet für ein Fluoraluminiumsilikatglasspulver, ein dentales Zinkphosphat-Zementpulver oder ein dentales Carboxylat-Zementpulver, und
0,01 bis 5 Gewichts-% von der Gesamtheit aus einer organischen aromatischen Verbindung, die mindestens eine -SO₂- Gruppe enthält, und einem Peroxid als ein Polymerisationskatalysator.

2. Zahnzement nach Anspruch 1, wobei das flüssige Mittel weiter 0,1 bis 25 Gewichts-% Wasser umfasst.

3. Zahnzement nach Anspruch 2, wobei ein Monomer mit einer Carboxylgruppe als ein (Meth)acrylat-Monomer mit einer Säuregruppe in dem flüssigen Mittel eingeschlossen ist.

## Revendications

1. Un ciment dentaire comprenant :
un agent liquide comprenant
de 5 à 50% en poids d'un monomère de (méth)acrylate ayant un groupe acide,
de 30 à 85% en poids d'un monomère de (méth)acrylate ayant deux ou moins de groupes hydroxyles et/ou de groupes amino, n'ayant pas de groupe acide, et ayant un poids moléculaire de 160 ou plus, et
de 0,01 à 5% en poids d'un composé amine en tant qu'initiateur de polymérisation d'un catalyseur de polymérisation dans un agent en poudre décrit ci-dessous ; et un agent en poudre comprenant de 90 à 99,8% en poids d'une poudre d'oxyde de métal utilisée pour une poudre de verre de fluoroaluminosilicate, une poudre de ciment dentaire de phosphate de zinc, ou une poudre de ciment dentaire de carboxylate,
et
de 0,01 à 5% en poids du total d'un composé aromatique organique contenant au moins un groupe -SO₂- et un peroxyde en tant que catalyseur de polymérisation.

2. Le ciment dentaire selon la revendication 1, dans lequel l'agent liquide comprend en outre de 0,1 à 25% en poids d'eau.

3. Le ciment dentaire tel que revendiqué à la revendication 2, dans lequel un monomère ayant un groupe carboxyle est inclus en tant que monomère de (méth)acrylate ayant un groupe acide dans l'agent liquide.
